# EUROPEAN PATENT SPECIFICATION

(11) **EP 1 010 435 B1**
(45) Date of publication and mention of the grant of the patent: **03.11.2010**
(21) Application number: 98941713.4
(22) Date of filing: 04.09.1998
(51) Int. Cl.: A61K 47/30, A61K 9/51

(54) **NANOCAPSULE PREPARATIONS FOR TREATING INTRAARTICULAR DISEASES**
ZUSAMMENSETZUNG AUS NANOKAPSELN ZUR BEHANDLUNG VON INTRAARTICULAEREN ERKRANKUNGEN
PREPARATIONS DE NANOCAPSULES DESTINEES AU TRAITEMENT DE MALADIES INTRA-ARTICULAIRES

(30) Priority: 05.09.1997 JP 24159897
(43) Date of publication of application: 21.06.2000
(73) Proprietor: Maruho K.K., Osaka-shi, Osaka 531-0071 (JP)
(72) Inventor: IMASATO, Yu, Toyonaka-shi, Osaka 565-0082 (JP); HORISAWA, Eiziro, Nishikasugai-gun, Aichi 452-0941 (JP); KAWAZOE, Satoko, Hikone-shi, Siga 522-0056 (JP); HIROTA, Tsuyoshi, Hikone-shi, Siga 522-0201 (JP); YAMADA, Jun, Hikone-shi, Siga 522-0054 (JP); KAWASHIMA, Yoshiaki, Gifu-shi, Gifu 502-0851 (JP); TAKEUCHI, Hirofumi, Gifu-shi, Gifu 502-0006 (JP); YAMAMOTO, Hiromitsu, Gifu-shi Gifu 502-0812 (JP)
(74) Representative: Plougmann & Vingtoft A/S
(86) International application number: PCT/JP1998/003966
(87) International publication number: WO 1999/012571

(56) References cited:
- EP-A- 0 896 825
- EP-A1- 0 635 261
- WO-A-93/05768
- WO-A-96/14830
- WO-A-96/31202
- WO-A1-87/02703
- WO-A1-95/03036
- CA-A1- 2 062 303
- JP-A- 5 105 628
- JP-A- 5 139 954
- JP-A- 5 255 077
- US-A- 4 530 840
- FESSI H ET AL: "NANOCAPSULE FORMATION BY INTERFACIAL POLYMER DEPOSITION FOLLOWING SOLVENT DISPLACEMENT" INTERNATIONAL JOURNAL OF PHARMACEUTICS, AMSTERDAM, NL, vol. 55, no. 1, 1 October 1989 (1989-10-01), pages R01-R04, XP001080379 ISSN: 0378-5173
- HORISAWA ET AL: 'Size-Dependency of DL-Lactide/Glycolide Copolymer Particulates for Intra-Articular Delivery System on Phagocytosis in Rat Synovium' PHARMACEUTICAL RESEARCH vol. 19, no. 2, 02 February 2002, pages 132 - 139
- CALVO ET AL: 'Novel Hydropilic Chitosan-Polyethylene Oxide' JOURNAL OF APPLIED POLYMER SCIENCE vol. 63, 1997, pages 125 - 132

## Description

### FIELD OF ART

The present invention relates to use of
i) a physiologically active substance selected from the group consisting of an analgesic, an anti-inflammatory agent, a DMARD, a cartilage degradation Inhibitor, an immunomodulator, an immunosuppressant, an antiallergic agent, a bone resorption inhibitor, and a radical scavenger, and
ii) a biocompatible polymer which allows a sustained release of the physiologically active substance,
for the preparation of a nanocapsule preparation for intra-articular administration for treatment of an intra-articular
disorder, wherein the nanocapsules have an average particle diameter of 0.05 µm to 0.45 µm wherein:
the analgesic is selected from the group consisting of methyl salicylate, flufenamic acid, sulpyrin, morphine, pethidine, levorphanol tartrate, aspirin, phenacetin, sasapyrine, salicylamide and salts thereof;
the anti-inflammatory agent is selected from the group consisting of dexamethasone, triamcinolone, triamcinolone acetonide, halopredone, paramethasone, hydrocortisone, prednisolone, methyl prednisolone, betamethasone, and salts thereof; and
the DMARD is selected from the group consisting of auranofin, gold sodium thiomalate, methotrexate, bucillamine, D-penicillamine, lobenzarit disodium, actarit and sulfasalazine; and
the cartilage degradation inhibitor is selected from the group consisting of marimastat, TIMP, collagenase, stromelysin, elastase and cathepsin-G; and
the immunomodulator and immunosuppresant are selected from the group consisting of tacrolimus hydrate, cyclosporine, azathioprine, gusperimus hydrochloride and mizoribine; and
the antiallergic agent is selected from the group consisting of diphenylhydramine, chlorpheniramine, tripelennamine, methdilazine, clemizole, diphenylpyraline, methoxyphenamine, astemizole, amlexanox, ibudilast, ebastine, azelastine, hydrochloride, ozagrel hydrochloride, oxatomide, sodium cromoglicate, seratrodast, tazanolast, terfenadine, suplatast tosylate, tranilast, emedastine difumarate, ketotifen fumarate, pranlukast hydrate, perimolast potassium, repirinast, and salts thereof, and
the bone resorption inhibitor is aminomethylene biphosphoric acid, and wherein the surface of the nanocapsules is covered with chitosan, pullulan or dextran having a molecular weight of 2,000 to 200,000.

### BACKGROUND ART

Conventionally, as therapy for osteoarthritis involving arthritis and rheumatoid arthritis, there is known local therapy of injecting anti-inflammatory agents effective against them directly in articular cavities.

Although such therapy has a drawback of giving pain to patients at injection, the therapy has an extremely little effect on the whole body and have the merit of being local treatment of direct sites of pain. It has been shown that they exhibit an evidently higher pharmacological efficacy than therapies with oral medicines.

However, since the local therapy is carried out with use of injection preparations (injections) prepared by dissolving or suspending medicaments in aqueous solution for injection or in oily liquid for injection, the medicaments rapidly disappear from articular cavities that are administration sites. Furthermore, in view of the safety of patients, one therapeutic cycle is generally set to about one week at most.

Accordingly, desired is development of sustained-release or long lasting articular injection preparations in view of duration of effect and further in view of QOL (Quality of Life).

For example, S. S. Davis et al. have proposed an attempt to administer, to knee joints of rabbits, a corticoid which is micro-capsulated with use of rabbit serum albumin as a support carrier (J. Pharm. Pharmacol., 39, 290-295, (1987)). This reference describes that though the prolongation of drug efficacy is realized, there is room for further study in that the micro-capsules are taken in by macrophages because of their sizes, in that the micro-capsules cause damage to tissue because of their materials, and the like.

Further, Mizushima, Hoshi et al. have developed a variety of steroid-suspended preparations (suspensions of crystals of medicaments) (Arzneim.-Forsch / Drug Res., 30,(I), Nr 2, (1980); Nippon Rinsho, 47, (6), 1302-1307, (1989)). It has been reported that these preparations have good results in providing sustained release of medicaments locally. Especially, some of these preparations are used in practical medical situations and earn good reputation as preparations providing improved QOL.

Recently, however, a new problem of crystals-induced pain caused by these preparations in articular cavities is getting a great deal of attention. That is, the medicaments intended to serve for treatment also act as foreign substances and newly induce damage or pain in articular sites by remaining in articular cavities for a long time without dissolving.

The cause of the crystals-induced pain is not made clear. But, since the induction of pain has not been seen so often with conventional solution preparations, it is supposed or pointed out that physicochemical properties of particles of medicaments, i.e., the diameter and shape of particles and the like, closely relate to the induction, in addition to properties of particles of medicaments in suspensions, i.e., bio-incompatibility of suspended particles, doses and the like. Further, the prolonged existence of the crystals of medicaments in articular cavities is also considered to bring about accumulated damage to tissue.

Under these circumstances, there is an increasing need for a safe articular injection preparation that has a long lasting property and does not give pain to patients.

### DISCLOSURE OF THE INVENTION

The inventors of the present invention have done intensive studies, aiming at development of a long lasting preparation for treatment for arthritis which is free from the problem of crystal-induced pain to reduce pain to a patient.

That is, the inventors have considered that it might be possible to design a suitable preparation by placing a suspension of biocompatible fine particles containing a medicament in an articular cavity, and have done researches on the following necessary factors:
(1) a biocompatible base suitable for constituting nanocapsules enclosing a medicament;
(2) properties that nanocapsules should have for avoiding damage to tissue;
(3) a preparation formula suitable for releasing the medicaments from nanocapsules continuously; and the like,
finally to accomplish the present invention which relates to a safe preparation which has not existed so far and which can exhibit a long lasting property and reduce pain to patients.

According to the present invention, there is provided a nanocapsule preparation for intra-articular administration for treatment of an intra-articular disorder comprising a physiologically active substance and a biocompatible polymer allowing sustained release of the physiologically active substance.

### BRIEF DESCRIPTION OF THE DRAWINGS

Fig. 1 is a graphical representation showing results of the release of nanocapsulated medicaments contained in preparations in accordance with the present invention;
Fig. 2 is a graphical representation showing changes with time in the amount of a medicament remaining in an air pouch and the amount of the medicament eluted or dissolved in the air pouch when a sample containing nanocapsules (LA/GLA = 75/25, MW = 20,000) in accordance with the present invention is administered in the air pouch;
Fig. 3 is a graphical representation showing joint swelling (increase) of knees with time after booster in rabbits adjuvant arthritis models to which a sample containing nanocapsules (LA/GLA = 75/25, MW = 20,000) in accordance with the present invention is administered; and
Fig. 4 is a graphical representation showing the surface skin temperature (increase) of knees with time after booster in rabbits adjuvant arthritis models to which a sample containing nanocapsules (LA/GLA = 75/25, MW = 20,000) in accordance with the present invention is administered.

### BEST MODE FOR CARRYING OUT THE INVENTION

The present invention relates to a nanocapsule preparation for intra-articular administration for treatment of an intra-articular disorder comprising a physiologically active substance and a biocompatible polymer which allows sustained release of the physiologically active substance.

The "nanocapsules" in the present invention mean nanometer-sized fine capsules whose average particle diameter is 0.05 µm to 0.45 µm, using measurement by a laser diffraction dynamic light scattering method. The shape of capsules is spherical or approximately spherical. With such a size and shape, that is, where the nanocapsules are fine and spherical, the nanocapsules have a smaller surface area than crystals of medicaments which generally vary in shape. Accordingly, the recognition by living bodies of the nanocapsules in articular cavities as foreign substances owing to their size and shape can be decreased, and various undesirable reactions which may result in damage to tissue can be inhibited.

The nanocapsules of the present invention are composed basically of a physiologically active substance and a biocompatible polymer. Preferably, fine particles of the physiologically active substance are substantially dispersed in the biocompatible polymer if observed by a scanning electron microscope. It is not a requirement that all the fine particles of the physiologically active substance are dispersed in the biocompatible polymer. But, since one of the major objects is that the physiologically active substance is released continuously in a living body by being nanocapsulated, it is preferable from this viewpoint that the fine particles of the physiologically active substance are dispersed in the biocompatible polymer at least physically.

The physiologically active substance in the present invention is selected from the group consisting of an analgesic, an anti-inflammatory agent, a DMARD, a cartilage degradation inhibitor, an immunomodulator, an immunosuppressant, an antiallergic agent, a bone resorption inhibitor and a radical scavenger.

The analgesic, is selected from the group consisting of methyl salicylate, flufenamic acid, sulpyrin, morphine, pethidine, levorphanol tartrate, aspirin, phenacetin, sasapyrine, salicylamide and salts thereof.

The anti-inflammatory agent is selected from the group consisting of dexamethasone, triamcinolone, triamcinolone acetonide, halopredone, paramethasone, hydrocortisone, prednisolone, methyl prednisolone, betamethasone.

The DMARDs is selected from the group consisting of auranofin, gold sodium thiomalate; methotrexate; bucillamine; D-penicillamine; lobenzarit disodium; actarit; and sulfasalazine.

The cartilage degradation inhibitor, is selected from the group consisting of marimastat, TIMP, collagenase, stromelysin, elastase and cathepsin-G.

The immunomodulator and immunosuppressant, are selected from for the group consisting of tacrolimus hydrate, cyclosporine, azathioprine, gusperimus hydrochloride, and mizoribine.

The antiallergic agent, is selected from the group consisting of diphenhydramine, chlorpheniramine, tripelennamine, methdilazine, clemizole, diphenylpyraline, methoxyphenamine, astemizole, amlexanox, ibudilast, ebastine, azelastine hydrochloride, ozagrel hydrochloride, oxatomide, sodium cromoglicate, seratrodast, tazanolast, terfenadine, suplatast tosylate, tranilast, emedastine difumarate, ketotifen fumarate, pranlukast hydrate, pemirolast potassium, repirinast, and salts thereof.

The bone resorption inhibitor, is aminomethylene bisphosphoric acid.

As biocompatible polymers of the present invention, known natural or synthetic polymers may be mentioned. Preferably, the polymers are also biodegradable. Specifically, may be mentioned homopolymers comprised of lactic acid, glycolic acid, butyric acid, hydroxybutyric acid and oxalic acid (for example, poly-d,l-lactic acid, polyglycolic acid, poly- β - hydroxybutylate (PHBA), poly-p-dioxane (PDS), polyesteramide, polyester of oxalic acid, poly- ε-caprolactone, etc.); copolymers (glycollide / L lactide copolymer, PHBA/ β -hydroxyvalerate copolymer, etc.); mixtures of two or more homopolymers thereof; mixtures of homopolymers and copolymers thereof; mixtures of two or more copolymers thereof and the like. As for optically active substances, any of d-form, l-form and mixture thereof may be used.

These polymers preferably have a molecular weight of about 2,000 to about 500,000, more preferably about 2,000 to about 200,000, still more preferably about 5,000 to about 100,000.

Among these polymers, it is preferable to use poly-d,l-lactic acid, polyglycolic acid or d,l-lactic acid/glycolic acid copolymer having a molecular weight of about 2,000 to about 200,000. In the case of mixtures of homopolymer(s) and/or copolymer(s), in particular, poly-d,l-lactic acid having a molecular weight of about 5,000 to about 100,000 is preferably blended in a ratio of about 5% or more.

Preferably, the nanocapsules of the present invention contain about 0.01% to about 75% (w/w), more preferably about 0.01% to about 20% (w/w), of the physiologically active substance therein.

The nanocapsules of the present invention may be prepared by various methods including a solvent diffusion method (for example, Japanese Patent Application Laid-Open No. HEI 5(1993)-58882), a phase separation method, a submerged drying method, a spray drying method, a freeze grinding method, which are known microcapsulation techniques. Among these methods, the solvent diffusion method (in-oil method or in-water method) and phase separation method are preferred. Additionally, as regards the solvent diffusion method, which to select to use between the solvent-diffusion-in-oil method and the solvent-diffusion-in-water method can be determined as appropriate depending upon the solubility of the physiologically active substance or the like.

For example, the nanocapsules of the present invention may be prepared by the solvent-diffusion-in-oil method (O / O method) in the following manner:

First, the physiologically active substance and the biocompatible and preferably biodegradable polymer are dissolved in an organic solvent to prepare a solution. Next, the solvent is added with stirring into an oil phase to which both the physiologically active substance and the polymer are insoluble.

As organic solvents usable here, for example, may be mentioned lower alcohols such as ethanol and methanol; acetone, acetonitrile, dichloromethane, chloroform, and mixture liquids thereof. Among these solvents, it is preferable to use a mixture of acetone and a lower alcohol in an appropriate mixture ratio (for example, about 50 : 1 to 1 : 1). Further, for the oil phase, it is preferable to use an oil whose polarity is low. For example, may be mentioned hydrocarbons such as liquid paraffin and squalane, mono-, di- or tri-glycerides of medium-chain fatty acids (for example, caprylic acid, capric acid, lauric acid, laurylic acid, myristic acid, etc.), esters of medium-chain fatty acids and alcohols (for example, isopropyl myristate, hexyl laurate, hexyldecyl isostearate, etc.), higher alcohols (for example, oleyl alcohol, isohexyldecanol, etc.) and organic solvents such as n-hexane, among which glycerides of medium-chain fatty acids are preferred. In addition, one species or two or more species of nonionic, anionic or cationic surfactants (for example, polyoxyethylene, polyoxypropylene, polysorbates, polyoxyethylene hydrogenated castor oils, sorbitan fatty acid esters, polyvinylpyrrolidone, etc.) may be added to the organic solvent and/or the oil phase.

When the solvent containing the physiologically active substance and the biocompatible and preferably biodegradable polymer is added to the oil phase as described above, the organic solvent in the solution diffuses in the oil phase immediately, and nanocapsules are precipitated which are fine spherical particles of the polymer enclosing the physiologically active substance.

Incidentally, when the solution is added to and/or stirred in the oil phase, pressure may optionally be reduced to about 700 mmHg or lower, or the oil phase may be heated to a temperature lower than the glass transition point of the polymer. Further, the rate of adding the solution is preferably about 0.5 ml/minute to about 10 ml/minute.

The thus obtained nanocapsules can be separated by centrifugal operation or by filtering operation. Thereafter, the nanocapsules may optionally be washed repeatedly several times with n-hexane, distilled water, distilled water to which a surfactant (for example, polyvinyl alcohol, polyvinylpyrrolidone, carboxymethyl cellulose, etc.) is added, or the like for the purpose of removing the surfactant or the like adhered to the surface of the nanocapsules. Further, the washed nanocapsules may be redispersed in distilled water and then freeze-dried. In this connection, when the nanocapsules are dispersed again in distilled water, an additive such as mannitol may optionally be added with a view to preventing aggregation.

Alternatively, the nanocapsules of the present invention may be prepared by the solvent-diffusion-in-water method (O/W method) in the following manner:

As in the above-described solvent-diffusion-in-oil method, the physiologically active substance and the biocompatible and preferably biodegradable polymer are dissolved in an organic solvent to prepare a solution. Next, this solution is added with stirring into an aqueous phase to which both the substance and the polymer are insoluble.

As organic solvents usable here, the same solvents as mentioned above may be mentioned. Further, a surfactant may be added to the organic solvent as described above. For the aqueous phase, distilled water is usually used, to which a surfactant as mentioned above may be added in a ratio of about 0.01 % to about 10% (w / w). Particularly, preferable is distilled water to which polyvinyl alcohol is added in a concentration of about 2%.

As described above, when the solution containing the physiologically active substance and the biocompatible and preferably biodegradable polymer is added into the aqueous phase, the organic solvent in the solution diffuses in the aqueous phase immediately, and nanocapsules are precipitated which are fine spherical particles of the polymer enclosing the physiologically active substance.

Incidentally, when the solution is added to and/or stirred in the water phase, pressure may be reduced or temperature may be raised, as described above. The rate of adding the solution may be the same as described above. The obtained nanocapsules may also be washed, dispersed again and freeze-dried.

Further, in the case where the nanocapsules of the present invention are prepared by the submerged drying method (W / O / W method), they may be prepared as follows:

First, the physiologically active substance is dissolved in a small amount of distilled water (for example, about 1% to about 25% with respect to a polymer solution) to prepare an aqueous solution. This aqueous solution is added to a solution which has been obtained by dissolving the biocompatible or biodegradable polymer and optionally a surfactant in an organic solvent, thereby to prepare an emulsion. The obtained emulsion is prepared into fine emulsified drops by emulsifying operation using a high-speed stir-shearing machine, a homogenizer or the like, by supersonic operation or the like. The emulsified drops are added to an aqueous phase containing a surfactant (for example, polyvinyl alcohol, polyvinyl pyrrolidone, carboxymethyl cellulose, etc.) to produce a W/O/W emulsion. Thereafter, the organic solvent is dried to obtain nanocapsules. The process afterward may be carried out as in the above-described solvent-diffusion-in-oil method or solvent-diffusion-in-water method.

The nanocapsule preparation of the present invention means an injection solution for injection, instillation or the like, or a preparation which is dissolved before use (The preparation may be nanocapsules themselves, or an appropriate additive may be optionally added, if necessary). Especially, the preparation is preferably in the form of an injection solution.

The nanocapsule preparation of the present invention may be adapted to have the form of an injection solution by dispersing the nanocapsules in an appropriate solvent and blending an appropriate additive and the like as required. The preparation to be dissolved before use can be used by being dispersed in an appropriate solvent before use.

As the appropriate solvent, any of those which do not affect living bodies and the physiologically active substance may be used. For example, distilled water for injection, sterilized purified water and the like may be mentioned, among which distilled water for injection is preferred. The nanocapsules are preferably dispersed in the solvent, for example, in distilled water for injection, in a ratio of about 0.01% to about 75% (w / w), more preferably about 1% to about 50% (w/w), still more preferably about 5% to about 25% (w/w).

As appropriate additives, additives usually used in this field of art may be mentioned, for example, dispersants (for example, Tween 80, carboxymethyl cellulose, protamine sulfate, polyethyleneglycol 400, etc.), preservatives (for example, methylparaben, propylparaben, etc.), isotonizers (for example, sodium chloride, mannitol, etc.), local anesthetics (for example, xylocain hydrochloride, chlorobutanol, etc.) and the like.

Further, the surface of the nanocapsules is covered with chitosan, pullulan or dextran, having a molecular weight of 2,000 to 200,000. Thereby, the adhesion of the nanocapsules to inflammatory cells or the transfer of the nanocapsules into inflammatory cells proceeds effectively, and the effect of the physiologically active substance can be enhanced further.

The chitosan, pullulan or dextran is preferably used within the range of about 0.001% to about 10% (w/w), more preferably about 2% or less, with respect to the nanocapsules.

As a method for covering the surface of nanocapsules with chitosan, pullulan or dextran, for example, may be mentioned a method of adding the chitosan, pullulan or dextran to a washing liquid in a concentration, for example, of 0.005% to 5% (w / w) when the prepared nanocapsules are washed. Also, as a method for making the chitosan, pullalan or dextran present in the nanocapsules or on the surface thereof, may be mentioned a method of adding the chitosan, pullalan or dextran to the solvent for precipitating the nanocapsules in a concentration, for example, of 0.2% to 2.5% (w/w).

The nanocapsules of the present invention are to be administered into joints of mammals including human: Here, the administration into joints means dispersing the nanocapsules of the present invention in an appropriate solvent and injecting them intra-articularly, usually in articular cavities or in vicinity thereof.

The dose of the nanocapsule preparation varies depending upon the kind and content of the physiologically active substance, the kind of a disease to be cured, the condition of the disease, the age of a patient and the like. However, the dose is required at least such that an effective concentration of the physiologically active substance is retained. Usually, the physiologically active substance may be administered at a dose of about 0.0001 mg/kg to about 100 mg/kg once in a day to once in a month per an adult.

The nanocapsule preparation for treatment of intra-articular diseases of the present invention is now described in detail with reference to examples and test examples.

### Example 1

(1) 10 mg of betamethasone sodium phosphate(BSP) and 100 mg of lactic acid-glycolic acid copolymer (PLGA7520 produced by Wako Junyaku, Japan)(LA/GLA = 75/25, MW = 20,000) were weighed precisely, and a polymer-medicament solution containing 3 ml of acetone, 0.3 ml of methanol and 100 mg of sorbitan monooleate (a surfactant, Span 80) was prepared. In a vessel provided with a stirring motor, the obtained solution was poured into 60 ml of caprylic acid-capric acid triglyceride (Triester F-810 produced by Nikko Chemicals, Japan) containing 2% of polyglyseryl-6-polyricinolate (Hexaglyn PR-15 produced by Nikko Chemicals), at a constant rate of 2 ml/minute with use of a peristaltic pump with stirring at 400 rpm.

The resulting solution was centrifuged at 20,000 rpm and the supernatant was disposed of. The residue (nanocapsules) was dispersed again by adding a small amount of n-hexane. Thereafter, centrifugation was again conducted under the same conditions. After the completion of the centrifugation, n-hexane was disposed of, and the residue was sufficiently dried.

A small amount of a 2% aqueous solution of polyvinyl alcohol was added to the dried residue to disperse it again. Another centrifugation was conducted. After completion of the centrifugation, a small amount of water was added to the obtained residue to disperse it again. The resulting liquid was freeze-dried to obtain nanocapsules.

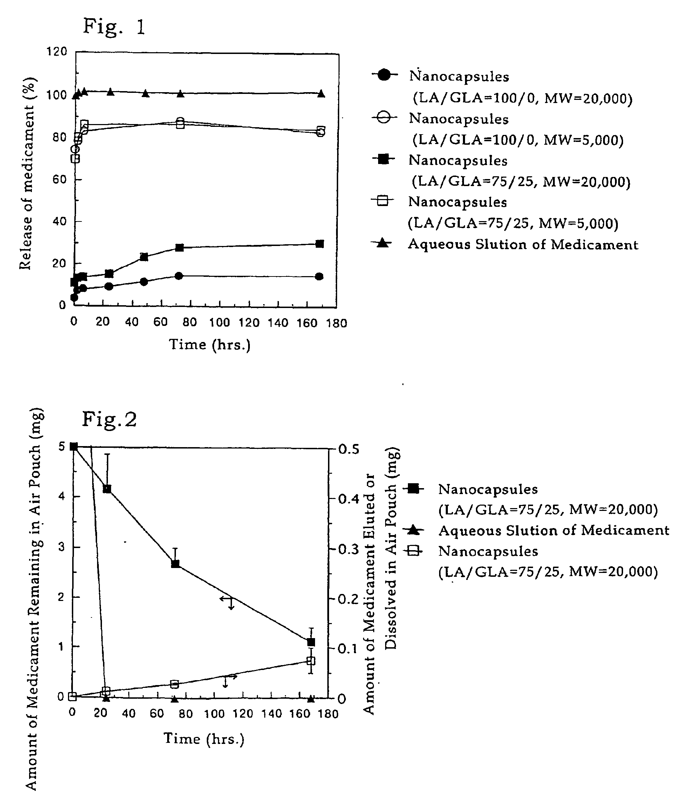

Three kinds of nanocapsules were also obtained by the same method as described above by use of polymers whose molecular weights and copolymerization ratios of lactic acid to glycolic acid are varied within the range shown in Fig. 1.

The average particle diameter of the obtained four kinds of nanocapsules was determined by a laser-diffraction particle size distribution analyzer. As a result, the average particle diameter was about 350 nm for all the kinds of nanocapsules. Electron-microscopic observation of the nanocapsules confirmed that all particles were 1 µm or smaller and spherical.

### Test Example 1

The nanocapsules (LA/GLA = 75/25, MW = 20,000) obtained in Example 1, 40 mg, was added to 16 ml of a pre-prepared isotonic phosphoric acid buffer (pH = 7.2). The mixture was oscillated at 37°C while measuring the amount of betamethasone sodium phosphate released into the solution with time. The nanocapsules shown in Fig. 1 which were different in the molecular weight and the copolymerization ratio of lactic acid to glycolic acid were also tested in the same way.

The results are shown in Fig. 1.

According to Fig. 1, the nanocapsules of low-molecular polymers (LA/GLA = 100/0 and 75/25, MW = 5,000) each exhibited a medicament elution of 80% or more into the solution in several hours after the test was started. However, the nanocapsules of high-molecular polymers (LA/GLA = 100/0 and 75/25, MW = 20,000) exhibited a medicament elution of 30% or less even when 160 hours elapsed after the test was started. At that time, the nanocapsules of LA/GLA = 100/0 exhibited an medicament elution of about 15% and that of LA/GLA = 75/25 exhibited an medicament elution of about 30%. The higher the copolymerization ratio of GLA was, the higher the medicament elution became.

It has been gathered from the above results that the betamethasone phosphate elution characteristics of the nanocapsule preparations according to the present invention depend upon the molecular weight and the copolymerization ratio of GLA, that the higher the molecular weight, the lower the elution property can be suppressed, and that the higher the copolymerization ratio of GLA, the higher the elution property becomes. Accordingly, it is possible to control the medicament elution utilizing the molecular weight and the copolymerization ratio of GLA.

Especially, with the nanocapsules of high-molecular polymers (LA/GLA = 100/0 and 75/25, MW = 20,000), about 70% to about 85% of the medicament remain after 160 hours elapse. If they are administered in vivo, they are expected to exhibit a good long lasting property in vivo with additional elution of the medicament due to physiological degradation of the polymers.

### Test Example 2

The nanocapsules obtained in Example 1 (LA/GLA = 75/25, MW = 20,000), about 80 mg (the amount containing 5 mg of the medicament (BSP)), was dispersed in 1 ml of physiological saline to prepare a suspension, which was used as a sample of the present invention.

Air pouches of about 10 ml were formed on the backs of rats according to the method of Mizushima et al (for example, Arzneim.-Forsch / Drug Res., 30, (I), Nr 2, (1980)).

Penicillin 10,000 IU 10 mg was dissolved in a 2% aqueous solution of carboxymethyl cellulose sodium so that the total amount became 6 ml, which was injected into an air pouch with use of a syringe provided with an injection needle 24 hours after the air pouch was formed. After another 24 hours, 0.5 ml of a liquid prepared by dissolving lipopolysaccharide in physiological saline in a concentration of 10 ng / ml were injected. After another hour, the sample of the present invention was administered into the air pouch using a syringe provided with an injection needle.

The pouch was opened 24, 72 and 168 hours after the administration. Liquid inside the pouch was washed with physiological saline and part of wash liquid was completely dissolved with acetone.

Thereafter, the amount of betamethasone sodium phosphate remaining in the inside liquid was measured.

Further, in order to determine the amount of the medicament dissolved in the air pouch, part of the wash liquid was taken out in such a manner that remaining fine particles were not dissolved, and centrifuged. The supernatant was filtered with a 0.2 µm membrane filter. The resulting liquid was subjected to a quantitative test.

In addition, as a control, an aqueous solution of betamethasone sodium phosphate was prepared so that the amount of the medicament (BSP) was ten times larger, 50mg, and administered into an air pouch similarly. Also, as a blank test, physiological saline was administered into an air pouch of a rat.

The obtained results are shown in Fig. 2.

According to changes with time in the amount of the medicament remaining in the air pouches, it was found that, with the aqueous solution of betamethasone sodium phosphate (a medicament aqueous solution) corresponding to a ten-time larger dose, the medicament already disappeared when 24 hours elapsed after administration, while, with the sample containing the nanocapsules of Example 1, the amount of the remaining medicament decreased gradually after administration and about 20% of the initially administered betamethasone sodium phosphate remained in the air pouch when 168 hours elapsed.

Also, according to changes with time in the amount of the medicament dissolved or eluted in the air pouches shown in Fig. 2, it was found that betamethasone sodium phosphate dissolved or eluted in the air pouch increased gradually after administration.

Further, after 168 hours elapsed, the air pouches were opened and subcutanous and intracutaneous parts were observed with the naked eye. Compared with the blank test, there were not found abnormal conditions such as changes in the inside liquid and penetrating substances which may cause inflammation or such as flare and the like which may be caused by symptoms such as infiltration or growth of subcutanous and intracutaneous cells.

It has been found from the above-mentioned that the nanocapsule preparation of the present invention are safe and remain in living body cavities for a long time.

### Test Example 3

The nanocapsules (LA/GLA = 75/25, MW = 20,000) obtained in Example 1, about 50 mg (an amount containing 3 mg of the medicament (BSP)), was dispersed in 250 µl of physiological saline to prepare a suspension, which was used as a sample of the present invention.

Produced were rabbit models in which adjuvant arthritis was induced with ovalbumin (FCA) according to the method of E.R.Pettipher et al. (Br.J.Exp.Path., 69, 113-122, (1988)). Three weeks after sensitization, the models were boosted again with ovalbumin at knees. Simultaneously with boosting, the sample of the present invention was administered in articular cavities.

An aqueous solution of betamethasone sodium phosphate so prepared that the amount of the medicament (BSP) was the same, 3mg, and physiological saline alone were administered in articular cavities as a control and as a blank test, respectively, simultaneously with boosting.

The outside diameter of the knees of rabbits were measured with a caliper 1 day, 3 days, 7 days, 14 days, 21 days, 28 days, 35 days and 42 days after administration. Skin temperature at the knees was measured with a contact-type surface thermometer.

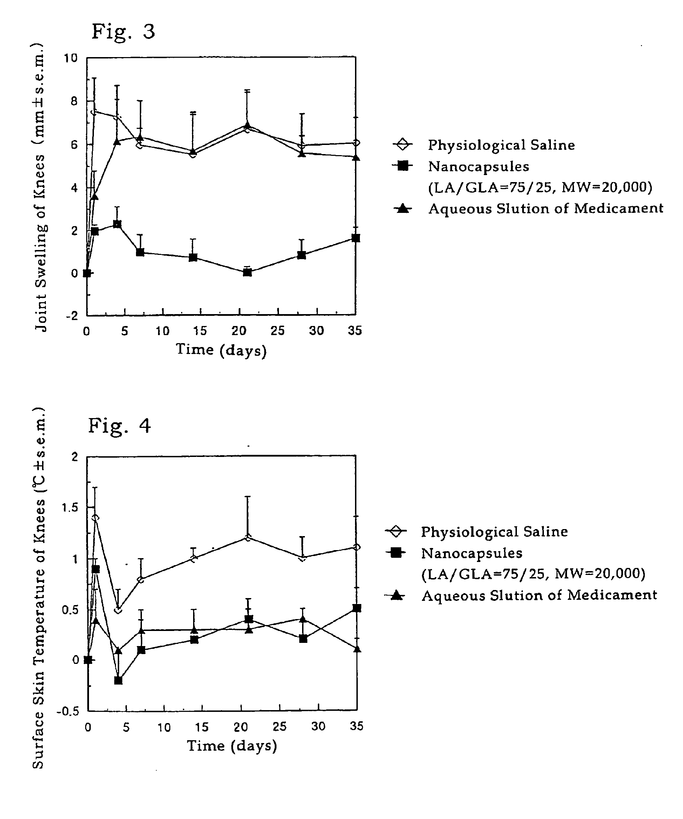

The results are shown in Figs. 3 and 4.

The outside diameter of the knees and temperature measured with time are shown as values obtained by subtracting reference values from the measured values. The reference values are those before the boosting at the knees.

It is generally known that the swell of knees reaches it maximum 24 hours after boosting and is accompanied by fever (E.R.Pettipher, G.A.Higgs and B.Henderson., Agents Action, 21, 98-103, (1987)). However, Fig. 3 shows that in the case where the sample of the present invention was administered, the swell of knees was significantly suppressed since one day had passed after administration and the swell was continuously suppressed even after 5 weeks. On the other hand, in the case where the aqueous solution of betamethasone sodium phosphate was administered, the swell of knees was suppressed one day after administration. However, the swell gradually increases after that and almost the same swell as in the case of physiological saline was observed.

Also, Fig. 4 shows that the case of administering the sample of the present invention was not different from the case of administering the aqueous solution betamethasone sodium phosphate with regard to the temperature of knees, and therefore in both the cases, fever due to inflammation was suppressed.

Further, blood was collected from rabbits 2 weeks, 4 weeks and 6 weeks after boosting, and the production of antibodies for ovalbumin was mesured by the ELISA method (according to the same reference by E.R.Pettipher). In addition, 6 weeks after boosting, the knee joints of rabbits are opened, and the inside of the articular cavities and surrounding tissue were morphologically observed. The results showed that the infiltration and the like of inflammatory cells which were typical conditions reduces in joints to which the sample of the present invention was administered and confirmed suppression in the production of antibodies.

### Example 2

Lysozyme hydrochloride, 10 mg, and histidine, 1 mg, were weighed and dissolved in 0.2 ml of purified water. This solution was added to a liquid in which 100 mg of polylactic acid (MW = 20,000) and 100 mg of sorbitan monooleate had been dissolved beforehand in 2 ml of methylene chloride. The resulting solution was stirred at 18,000 rpm for 2 minutes by a stirrer to produce a W / O emulsion.

In a vessel provided with a stirring motor, the above-obtained emulsion containing lysozyme hydrochloride was poured into 100 ml of caprylic acid-capric acid triglyceride (Triester F-810) containing 2% of polyglyseryl-6-polyricinolate (Hexaglyn PR-15), at a constant rate of 2 ml / minute with use of a peristaltic pump with stirring at 600 rpm. Thereafter, the mixture was stirred for another 2 hours under reduced pressure. After stirring, the resulting liquid was centrifuged at 20,000 rpm and the supernatant was disposed of to obtain the residual (nanocapsules). Further, the residue was dispersed in a 2% aqueous solution of polyvinyl alcohol and centrifuged again. The resulting residue was dispersed again by adding a small amount of water and the resulting liquid was freeze-dried to obtain nanocapsules.

Electron-microscopic observation of the obtained nanocapsules showed that the average particle diameter was smaller than 1 µm.

### Example 3

Mucopolysaccharide, 3 g, and arginine, 300 mg, were weighed and dissolved in 10 ml of phosphoric acid buffer. This solution was added to a liquid in which 200 g of lactic acid/glycolic acid copolymer (LA/GLA = 50/50, MW = 100,000) and 100g of sorbitan monooleate had been dissolved beforehand in 60 ml of chloroform. Thereafter, the resulting solution was stirred at 20,000 rpm for 2 minutes with a stirrer to produce a W/O emulsion.

In a vessel provided with a stirring motor, the above-obtained liquid containing the W / O emulsion was poured into 2,000 ml of a 2% aqueous solution of polyvinyl alcohol (pH = 5.0), at a constant rate of 10 ml/minute with use of a tube pump with stirring at 400 rpm. Thereafter, the mixture was stirred for another 2 hours under reduced pressure. After stirring, the resulting liquid was centrifuged at 20,000 rpm and the supernatant was disposed of to obtain the residue (nanocapsules). The residue was further dispersed in water and centrifuged again. The resulting residue was dispersed again by adding water and the resulting liquid was freeze-dried to obtain nanocapsules.

Electron-microscopic observation of the obtained nanocapsules showed that the average particle diameter was smaller than 1 µm.

### Example 4

A matrix metalloproteinase inhibitor, 1 mg, and lactic acid/glycolic acid copolymer (LA/GLA = 75/25, MW = 50,000), 100 mg, were precisely weighed and completely dissolved in 3 ml of a solution containing ethanol and acetone (1 : 2). The resulting liquid was dispersed in 100 ml of a 0.01% aqueous solution of polyvinyl alcohol with stirring by a stirrer. Thereafter, this dispersion was spray-dried under the conditions of a drying temperature of 50°C and a liquid sending rate of 5 ml/minute to obtain nanocapsules.

Electron-microscopic observation of the obtained nanocapsules showed that the average particle diameter was smaller than 1 µm.

### Example 5

Indomethacin, 15 mg, and polyglycolic acid (MW = 100,000), 100 mg, were precisely weighed and completely dissolved in 2 ml of a solution containing methanol and acetone (1 : 2). The obtained solution was dispersed in 60 ml of a 2% aqueous solution of polyvinyl alcohol with stirring by a stirrer. Thereafter, this dispersion was spray-dried in the same manner as in Example 4 to obtain nanocapsules.

Electron-microscopic observation of the obtained nanocapsules showed that the average particle diameter was smaller than 1 µm.

### Example 6

Betamethasone sodium phosphate, 10 mg, and lactic acid-glycolic acid copolymer (LA/GLA = 75/25, MW = 20,000) preliminarily labeled with FITC (fluorescenin 5-isothiocyanate), 100 mg, were precisely weighed and completely dissolved to prepare a polymer-medicament solution containing 3 ml of acetone, 0.3 ml of methanol and 100 mg of sorbitan monooleate (a surfactant, Span 80). In a vessel provided with a stirring motor, the obtained solution was poured into 60 ml of caprylic acid-capric acid triglyceride containing 2% of polyglyseryl-6-polyricinolate at a constant rate of 2 ml / minute by use of a peristaltic pump with stirring at 400 rpm.

The obtained solution was centrifuged at 20,000 rpm and the supernatant was disposes of. The resulting residue was dispersed again by adding a small amount of n-hexane and then centrifuged again under the same conditions. After completion of centrifugation, n-hexane was disposed of, and the residue was dried sufficiently.

The dried residue was dispersed by adding a 2% aqueous solution of polyvinyl alcohol which was so prepared that the final concentration of chitosan (MW = 50,000) dissolved using an acetic acid buffer of pH 4.4 was 0.2% (w/w). The resulting mixture was subjected to a 10 seconds' supersonic treatment and then centrifuged again. After completion of centrifugation, the obtained residue was dispersed again by adding a small amount of water and freeze-dried to obtain FITC nanocapsules coated with chitosan.

These chitosan-coated FITC nanocapsules were dispersed in a small amount of water and measured by a laser-diffraction particle size distribution analyzer. The results showed that the average particle diameter was 352 nm. The results of further measurement by a scanning electron microscope (SEM) showed that the average particle diameter was smaller than 1 µm.

Next, the chitosan-coated FITC nanocapsules were dispersed in hydrochloric acid-potassium phosphate buffers adjusted to pHs of 1 to 7. Electric charges on the surface of the nanocapsules was determined by a zeta potential meter (Zetamaster, ZEM5002). The results showed that as the pH shifted from around pH = 4 toward an acidic side, the zeta potential shifted to a positive charge (a positive side).

Thus, it was confirmed that chitosan covered the nanocapsules or adhered to the nanocapsules.

The chitosan-coated FITC nanocapsules and non-coated FITC nanocapsules as a control were added with use of physiological saline to confluent-cultivated human synovial cells in an amount of 500 µg/0.5 ml/well and incubated at 37°C under a condition of 5% of CO₂. The amount of FITC adhering to synovial cells, i.e., the amount of adhering nanocapsules was measured 1 hour, 2 hours, 3 hours and 4 hours after the beginning of incubation. The results showed that the non-coated FITC nanocapsules hardly adhered to synovial cells while 20% to 30% of the chitosan-coated FITC nanocapsules already adhered to synovial cells 1 hour after the beginning of incubation.

### Example 7

Mucopolysaccharide, 3 g, and arginine, 300 mg, were weighed and dissolved in 10 ml of phosphoric acid buffer. This solution was added to a liquid in which 200 g of lactic acid/glycolic acid copolymer (LA/GLA = 50/50, MW = 100,000) and 100 g of sorbitan monooleate had been dissolved beforehand in 60 ml of chloroform. Thereafter, the resulting solution was stirred at 20,000 rpm for 2 minutes by a stirrer to prepare a W / O emulsion.

In a vessel provided with a stirring motor, the above-obtained liquid containing the W / O emulsion was poured into 2,000 ml of a 2% aqueous solution of polyvinyl alcohol (pH = 5.0) in which chitosan (MW = 50,000) had been dissolved in a concentration of 2.5% (w/w), at a constant rate of 10 ml / minute with use of a tube pump with stirring at 400 rpm. Thereafter, the mixture was stirred under reduced pressure for 2 hours. After stirring, the obtained liquid was centrifuged at 20,000 rpm. The supernatant was disposed of to obtain the residue (nanocapsules). Further, the residue was dispersed in water and centrifuged again. The obtained residue was dispersed again by adding water. The resulting liquid was freeze-dried, and thus chitosan was allowed to be present in nanocapsules and on the surface thereof.

### Example 8

The nanocapsules obtained in Examples 2 to 7, 20 mg each, were dispersed in 1 ml of distilled water for injection thereby to prepare nanocapsule preparations.

### Example 9

The nanocapsules obtained in Examples 2 to 7, 20 mg each, were dispersed in 0.2 ml of a sterilized 0.05% aqueous solution of chitosan and 0.8 ml of distilled water for injection thereby to prepare nanocapsule preparations.

## Claims

1. Use of
i) a physiologically active substance selected from the group consisting of an analgesic, an anti-inflammatory agent, a DMARD, a cartilage degradation inhibitor, an immunomodulator, an immunosuppressant, an antiallergic agent, a bone resorption inhibitor, and a radical scavenger, and
ii) a biocompatible polymer which allows a sustained release of the physiologically active substance,
for the preparation of a nanocapsule preparation for intraarticular administration for treatment of an intra-articular disorder, wherein the nanocapsules have an average particle diameter of 0.05 µm to 0.45 µm wherein:
the analgesic is selected from the group consisting of methyl salicylate, flufenamic acid, sulpyrin, morphine, pethidine, levorphanol tartrate, aspirin, phenacetin, sasapyrine, salicylamide and salts thereof;
the anti-inflammatory agent is selected from the group consisting of dexamethasone, triamcinolone, triamcinolone acetonide, halopredone, paramethasone, hydrocortisone, prednisolone, methyl prednisolone, betamethasone, and salts thereof; and
the DMARD is selected from the group consisting of auranofin, gold sodium thiomalate, methotrexate, bucillamine, D-penicillamine, lobenzarit disodium, actarit and sulfasalazine; and
the cartilage degradation inhibitor is selected from the group consisting of marimastat, TIMP, collagenase, stromelysin, elastase and cathepsin-G; and
the immunomodulator and immunosuppresant are selected from the group consisting of tacrolimus hydrate, cyclosporine, azathioprine, gusperimus hydrochloride and mizoribine; and
the antiallergic agent is selected from the group consisting of diphenylhydramine, chlorpheniramine, tripelennamine, methdilazine, clemizole, diphenylpyraline, methoxyphenamine, astemizole, amlexanox, ibudilast, ebastine, azelastine, hydrochloride, ozagrel hydrochloride, oxatomide, sodium cromoglicate, seratrodast, tazanolast, terfenadine, suplatast tosylate, tranilast, emedastine difumarate, ketotifen fumarate, pranlukast hydrate, perimolast potassium, repirinast, and salts thereof, and
the bone resorption inhibitor is aminomethylene biphosphoric acid, and wherein the surface of the nanocapsules is covered with chitosan, pullulan or dextran having a molecular weight of 2,000 to 200,000.

2. The use according to claim 1, wherein the biocompatible polymer is a biodegradable polymer.

3. The use according to claim 1 or 2, wherein the biocompatible polymer is a homopolymer, copolymer or a mixture thereof which is obtained from a monomer selected from the group consisting of lactic acid, glycolic acid, butyric acid, hydroxybutyric acid and oxalic acid and which has an average molecular weight of 2,000 to 500,000.

4. The use according to any one of claims 1 to 3, wherein the physiologically active substance is contained in the nanocapsules in a ratio of 0.01% to 75% (w/w).

5. The use according to claim 4, wherein the physiologically active substance is contained in the nanocapsules in a ratio of 0.01% to 20% (w/w).

6. The use according to claim 4 or 5, wherein the high-molecular polysaccharide adherent to living bodies is within the range of 0.001% to 10% (w/w) with respect to the nanocapsules.

7. The use according to any one of claims 1 to 6, wherein the nanocapsule preparation is in the form of an injection to be prepared before use comprising nanocapsules and distilled water for injection.

## Patentansprüche

1. Verwendung von
i) einer physiologisch wirksamen Substanz, ausgewählt aus der Gruppe, bestehend aus einem Analgetikum, einem antiinflammatorischen Mittel, einem Basismedikament bei Rheuma (DMARD), einem Hemmer des Knorpelabbaus, einem Immunmodulator, einem Immunsuppressor, einem Antiallergikum, einem Hemmer der Knochenresorption und einem Radikalfänger, und
ii) einem biokompatiblen Polymer, das die retardierte Freisetzung der physiologisch wirksamen Substanz ermöglicht,
zur Herstellung eines Nanokapselpräparats zur intraartikulären Verabreichung zur Behandlung einer intraartikulären Störung, wobei die Nanokapseln einen durchschnittlichen Teilchendurchmesser von 0,05 µm bis 0,45 µm aufweisen, wobei:
das Analgetikum ausgewählt ist aus der Gruppe, bestehend aus Methylsalicylat, Flufenaminsäure, Sulpyrin, Morphin, Pethidin, Levorphanoltartrat, Aspirin, Phenacetin, Sasapyrin, Salicylamid und Salzen davon;
das antiinflammatorische Mittel ausgewählt ist aus der Gruppe, bestehend aus Dexamethason, Triamcinolon, Triamcinolonacetonid, Halopredon, Paramethason, Hydrocortison, Prednisolon, Methylprednisolon, Betamethason und Salzen davon; und
das DMARD ausgewählt ist aus der Gruppe, bestehend aus Auranofin, Goldnatriumthiomalat, Methotrexat, Bucillamin, D-Penicillamin, Lobenzarit-Dinatrium, Actarit und Sulfasalazin; und
der Hemmer des Knorpelabbaus ausgewählt ist aus der Gruppe, bestehend aus Marimastat, TIMP, Collagenase, Stromelysin, Elastase und Cathepsin-G; und
der Immunmodulator und der Immunsuppressor ausgewählt sind aus der Gruppe, bestehend aus Tacrolimushydrat, Cyclosporin, Azathioprin, Gusperimus-Hydrochlorid und Mizoribin; und
das Antiallergikum ausgewählt ist aus der Gruppe, bestehend aus Diphenylhydramin, Chlorpheniramin, Tripelennamin, Methdilazin, Clemizol, Diphenylpyralin, Methoxyphenamin, Astemizol, Amlexanox, Ibudilast, Ebastin, Azelastin-Hydrochlorid, Ozagrel-Hydrochlorid, Oxatomid, Natrium-Cromoglicat, Seratrodast, Tazanolast, Terfenadin, Suplatasttosylat, Tranilast, Emedastindifumarat, Ketotifenfumarat, Pranlukasthydrat, Perimolast-Kalium, Repirinast und Salzen davon, und
der Hemmer der Knochenresorption Aminomethylenbiphosphorsäure ist und wobei die Oberfläche der Nanokapseln mit Chitosan, Pullulan oder Dextran mit einem Molekulargewicht von 2.000 bis 200.000 bedeckt ist.

2. Verwendung nach Anspruch 1, wobei das biokompatible Polymer ein biologisch abbaubares Polymer ist.

3. Verwendung nach Anspruch 1 oder 2, wobei das biokompatible Polymer ein Homopolymer, Copolymer oder eine Mischung davon ist, das mit einem Monomer erhalten wird, ausgewählt aus der Gruppe, bestehend aus Milchsäure, Glycolsäure, Buttersäure, Hydroxybuttersäure und Oxalsäure, und das ein durchschnittliches Molekulargewicht von 2.000 bis 500.000 aufweist.

4. Verwendung nach einem der Ansprüche 1 bis 3, wobei die physiologisch wirksame Substanz mit einem Anteil von 0,01 bis 75 Gew.-% in den Nanokapseln enthalten ist.

5. Verwendung nach Anspruch 4, wobei die physiologisch wirksame Substanz mit einem Anteil von 0,01 bis 20 Gew.-% in den Nanokapseln enthalten ist.

6. Verwendung nach Anspruch 4 oder 5, wobei das hochmolekulare Polysaccharid, das an lebenden Körpern anhaftet, in Bezug auf die Nanokapseln im Bereich 0,001 bis 10 Gew.-% liegt.

7. Verwendung nach einem der Ansprüche 1-6, wobei das Nanokapselpräparat in Form einer Injektion umfassend Nanokapseln und destilliertes Wasser für Injektionszwecke vorliegt, die vor der Verwendung zubereitet wird.

## Revendications

1. Utilisation
i) d'une substance physiologiquement active choisie dans le groupe constitué par un antalgique, un agent anti-inflammatoire, un médicament de l'arthrite rhumatoïde, un inhibiteur de la dégradation du cartilage, un immunomodulateur, un immunosuppresseur, un agent antiallergique, un inhibiteur de la résorption osseuse et un piégeur de radicaux, et
ii) d'un polymère biocompatible qui permet une libération prolongée de la substance physiologiquement active,
pour la préparation d'une préparation de nanocapsules destinée à une administration intra-articulaire pour le traitement d'un trouble intra-articulaire, où les nanocapsules présentent un diamètre moyen de particule de 0,05 µm à 0,45 µm où :
l'antalgique est choisi dans le groupe constitué par le salicylate de méthyle, l'acide flufénamique, la sulpyrine, la morphine, la péthidine, le tartrate de lévorphanol, l'aspirine, la phénacétine, la sasapyrine, le salicylamide et des sels de ceux-ci ;
l'agent anti-inflammatoire est choisi dans le groupe constitué par la dexaméthasone, la triamcinolone, la triamcinolone acétonide, l'haloprédone, la paraméthasone, l'hydrocortisone, la prednisolone, la méthylprednisolone, la bétaméthasone, et des sels de celles-ci ; et
le médicament de l'arthrite rhumatoïde est choisi dans le groupe constitué par l'auranofine, l'aurothiomalate sodique, le méthotrexate, la bucillamine, la D-pénicillamine, le lobenzarit disodique, l'actarit et la sulfasalazine ; et
l'inhibiteur de la dégradation du cartilage est choisi dans le groupe constitué par le marimastat, un TIMP, la collagénase, la stromélysine, l'élastase et la cathepsine G ; et
l'immunomodulateur et l'immunosuppresseur sont choisis dans le groupe constitué par le tacrolimus hydraté, la cyclosporine, l'azathioprine, le chlorhydrate de guspérimus et la mizoribine ; et
l'agent antiallergique est choisi dans le groupe constitué par la diphénhydramine, la chlorphéniramine, la tripélénnamine, la méthdilazine, le clémizole, la diphénylpyraline, la méthoxyphénamine, l'astémizole, l'amléxanox, l'ibudilast, l'ébastine, le chlorhydrate d'azélastine, le chlorhydrate d'ozagrel, l'oxatomide, le cromoglycate de sodium, le sératrodast, le tazanolast, la terfénadine, le tosylate de suplatast, le tranilast, le difumarate d'émédastine, le fumarate de kétotifène, le pranlukast hydraté, le périmolast potassique, le répirinast, et des sels de ceux-ci, et
l'inhibiteur de la résorption osseuse est l'acide aminométhylène-biphosphorique, et où la surface des nanocapsules est couverte de chitosane, pullulane ou dextrane ayant une masse moléculaire de 2 000 à 200 000.

2. Utilisation selon la revendication 1, où le polymère biocompatible est un polymère biodégradable.

3. Utilisation selon la revendication 1 ou 2, où le polymère biocompatible est un homopolymère, un copolymère ou un mélange de ceux-ci qui est obtenu à partir d'un monomère choisi dans le groupe constitué par l'acide lactique, l'acide glycolique, l'acide butyrique, l'acide hydroxybutyrique et l'acide oxalique et qui a une masse moléculaire moyenne de 2 000 à 500 000.

4. Utilisation selon l'une quelconque des revendications 1 à 3, où la substance physiologiquement active est contenue dans les nanocapsules dans un rapport de 0,01 % à 75 % (p/p).

5. Utilisation selon la revendication 4, où la substance physiologiquement active est contenue dans les nanocapsules dans un rapport de 0,01 % à 20 % (p/p).

6. Utilisation selon la revendication 4 ou 5, où le polysaccharide de masse moléculaire élevée adhérant aux corps vivants se situe dans la plage de 0,001 % à 10 % (p/p) par rapport aux nanocapsules.

7. Utilisation selon l'une quelconque des revendications 1 à 6, où la préparation de nanocapsules est sous la forme d'une injection à préparer avant utilisation comprenant des nanocapsules et de l'eau distillée pour injection.
